# EUROPEAN PATENT SPECIFICATION

(11) **EP 0 369 741 B1**
(45) Date of publication and mention of the grant of the patent: **10.08.1994**
(21) Application number: 89311772.1
(22) Date of filing: 14.11.1989
(51) Int. Cl.: A61K 7/06, A61K 7/48, A61K 9/16

(54) **Cationic compositions for topical application**
Kationische Präparate zur topischen Anwendung
Compositions cationiques pour utilisation topique

(30) Priority: 16.11.1988 US 272600
(43) Date of publication of application: 23.05.1990
(73) Proprietor: Advanced Polymer Systems, Inc., Redwood City, CA 94063 (US)
(72) Inventor: Nacht, Sergio, Los Altos California 94022 (US); Won, Richard, Palo Alto California 94303 (US); Katz, Martin, Menlo Park California 94025 (US); Cheng, Tai, Mountain View California 94040 (US)
(74) Representative: Harrison, David Christopher

(56) References cited:
- EP-A- 0 285 694
- WO-A-88/01164
- COSMETICS & TOILETRIES, vol. 102,April 1987, pages 71-80, Allured Publishing Corp.; E.D. GODDARD: "Substantivitythrough cationic substitution"

## Description

The present invention relates generally to the preparation of topical compositions. More particularly, it relates to the preparation of cationic polymer delivery systems which prolong the activity of various topically active ingredients by increasing the substantivity on keratinic materials, such as hair and skin.

The adherent properties of topically-applied substances on hair and skin affects both initial adsorption and retention, particularly on subsequent exposure to water. The combined characteristics of adsorption and retention constitute the property referred to as "substantivity," which can be defined as the ability of a substance to be adsorbed onto keratin and to resist removal by water rinse-off.

An ideal topical substance would have adsorptive affinity for keratinic materials, retain activity for long periods of time, resist being washed away by perspiration and other contact with water, and be free of adverse interactions with other ingredients of which incorporation is desirous. No topical substance has yet been discovered which adequately satisfies all of these objectives.

Some of the most popular topically active preparations available in the market today include, for example, fragrance substances; cosmetic substances, such as lipsticks, make-up, and foundation powders; insect repellants; anti-bacterials; acne treatment formulations; hair treatment formulations, such as conditioners and hair growth promoting agents; and skin protection formulations, such as aging-prevention agents; and ultraviolet absorbing substances (sunscreens). These ingredients, substances, or formulations may be used alone, or in combination with each other, and may be applied in pure form or diluted in a suitable solvent or carrier.

A frequently observed problem with such topically active substances is the rapid loss of activity after application to the skin. Under usual conditions of heavy perspiration and/or contact with water, concentration of the above substances in their respective topical compositions is either diluted, thereby reducing effectiveness, or washed away, thereby losing all effectiveness. One way to extend activity is by increasing the concentrations of the active ingredient in their respective formulations. However, as concentrations are increased, so too are the risks of toxic and allergic reactions to the user. These reactions often occur with the higher concentrations, even if exposure to the product is relatively short.

A second drawback, unrelated to the safety of administration of such compositions, is the increased expense of using such compositions which are so easily washed away. For instance, to maintain an adequate level of protection from the sun, a sunbather would have to reapply sunscreen each time after entering the water and frequently after perspiring.

It would therefore be highly desirable to provide an approach for increasing the adsorptive affinity of topically active compositions to keratinic materials, and for prolonging the activity of such compositions, while simultaneously reducing the likelihood of toxic and/or allergic reaction to the user.

### Description of the Background Art

U.S. Patent No. 4,690,825 and EP-A-277211 (WO-A-8801164) disclose an uncharged polymer bead delivery system suitable for topical application. U.S. Patent No. 4,304,563 discloses cationic polymers (and methods for their preparation) useful as gels for treatment of keratinic materials, such as hair. European patent application 225615 discloses the use of cationic beads formed from a polystyrene sulfonate-divinyl benzene copolymer for the controlled oral delivery of negatively-charged drugs. South African patent application no. 872554 discloses sulfonic acid cationic ion exchange resin particles which have been impregnated with certain agents to enhance their suitability for oral drug delivery. U.S. Patent No. 3,691,270 discloses cosmetic compositions for the skin comprising microcapsules formed from an aveolar polymer, including polyvinylpyridine. The microcapsules, however, are uncharged. U.S. Patent No. 3,880,990 discloses orally-adminstratable compositions comprising drugs encapsulated in an anionic polymer. U.S. Patent No. 4,198,395, discloses a negatively-charged polymeric resin material which is useful for the treatment of hypercholesterolemia by oral administration. U.S. Patent No. 4,552,812, discloses the preparation of fluorescent and magnetic anionic beads useful in performing assays. European patent application 060 138 discloses the preparation of porous copolymeric blocks capable of absorbing and acting as a reservoir for liquids, such as perfume. European patent application 143 608 discloses a polymeric bead composition having a releasable lipophilic compound retained therein. British patent 1,482,663 describe polymeric bead compositions capable of holding water-soluble drugs. Cationic polymeric ion exchange resins, including styrene-divinylbenzene copolymers, are commercially available from suppliers such as Interaction Chemicals, Inc., Mountain View, California 94043; and Reilly Tar & Chemical Corp., Indianapolis, Indiana 46204. The ability of cationic materials to adsorb to the skin and hair is discussed in Goddard (1987) Cosmetics & Toiletries 102:71-80.

EP-A-277211 (mentioned above) is taken as the closest prior art. It describes non-charged impregnated particles in liquid vehicles as carriers.

### SUMMARY OF THE INVENTION

The present invention provides for the incorporation of topically active substances in a cationic polymer bead delivery system to form novel topical compositions. It has been found that the cationic functionality imparted on the surface of the polymeric bead surface enhances the substantivity of the topically active substances when the beads are applied to the skin or hair. Cationic polymeric delivery systems according to the present invention are keratinophilic compositions which exhibit an affinity for skin, hair, and other biological molecules. The cationic polymer bead delivery system comprises crosslinked polymer beads characterized by cationic functionalities on the bead surface, usually positively-charged pyridine and quaternary ammonium groups. The beads form a porous network capable of retaining large amounts of topically active substances. The cationic beads are non-collapsible, small diameter, having relatively large pores and a relatively high ratio of pore volume to bead volume.

The cationic polymer bead delivery system having active ingredients incorporated therein may be used as a topical product by itself or may be further incorporated into a carrier composition or other cosmetic product. When used alone, the cationic polymeric delivery system with incorporated topically active ingredient is a dry, free-flowing product which can be rubbed directly onto the skin, providing controlled release of the topically active ingredient over a prolonged period of time. In the more usual situation where the cationic polymeric delivery system is incorporated in other carriers, vehicles, solvents, or cosmetic preparations, use of this delivery system avoids incompatibilities, typically chemical or physical interactions, which might otherwise exist between the active ingredient and other ingredients in the topical preparation, or between the active substance and the carrier, vehicle, or solvent.

Virtually any physiologically-acceptable solvent or medium may be used as a carrier. To preserve the cationic functionality on the polymeric bead surface, however, the carrier should be at least slightly acidic is below pH6, preferably being below 5, and most preferably being in the range from 3 to 4. It will often be desirable to incorporate a physiologically-acceptable buffer in the carrier to maintain the pH within the range of interest.

The cationic polymer bead delivery system may be formed by suspension polymerization of suitable monomers, at least some of which include functionalities capable of carrying a positive charge under the conditions of use, in an immiscible phase including a porogen. Generally, the monomers and the porogen are first mixed together and the resulting mixture is then suspended in the immiscible phase, usually an aqueous phase. The immiscible phase is then agitated to form droplets of the monomer mixture, and polymerization of the monomer mixture is initiated to form the desired beads. Cationicity (i.e., cationic functionality) may be obtained by protonating (or quaternizing) the surface functionalities, e.g., a quaternary nitrogen in a pyridine or quaternary ammonium group, with an acid medium. Such protonation may be performed either before or after entrapping the active ingredient, depending on the conditions and the chemical characteristics of the particular ingredient sought to be incorporated.

Conveniently, the topically active substance may be used as a porogen in a one-step process where there will be no substantial degradation of the substance under the conditions of polymerization and the substance is otherwise suitable. Alternatively, for more labile ingredients (particularly those which are heat or radiation labile), the compositions of the present invention may be formed using a two-step process. In this process, the polymeric beads may be preformed using a substitute porogen, for example, an alkane, cycloalakane, or aromatic solvent. The beads are formed by suspension polymerization, as described above, and the substitute porogen is extracted from the resulting bead product. The desired active substance may then be introduced into the beads, typically by contact absorption, to create the desired product. Again, the polymeric beads may be protonated either before or after entrapping the topically active substance into the delivery system. In addition to allowing the incorporation of labile substances, such a two-step preparation process allows greater control over the structure of the bead based on a wider choice of porogen substances in reaction conditions, and thus may be the desired preparatory method even for less labile substances.

In addition to enhanced substantivity, active substances incorporated into the cationic polymer bead delivery system of the present invention have been found to provide enhanced effectiveness when compared to similar concentrations of the ingredient in non-cationic polymeric bead delivery systems. For example, sunscreen preparations incorporated into systems comprising cationic polymeric beads will be expected to have an enhanced SPF (Sun Protection Factor) rating when compared to otherwise identical preparations comprising a non-cationic polymer bead delivery system.

### DETAILED DESCRIPTION OF THE SPECIFIC EMBODIMENTS

The beads or microspheres used in connection with the present invention are rigid, open-pore, chemical and biologically inert particles, with a positive charge imparted on the surfaces and an impregnant held inside the pores by capillary forces, where the impregnant is a topically active substance and the charge is sufficient to promote adhesion of the particles to keratinic materials, such as human skin and hair. The pores are interconnected and open to the particle surface so that substantially full communication is provided between the internal pore space and the exterior of the particle whereby the impregnant may be released over time after the beads are applied to the user's skin or hair.

The cationicity of the polymeric beads derives from the presence of a functionality capable of being protonized on at least some of the monomers being polymerized. The functionalities of particular interest to the present invention include both pyridine and ammonium each of which have a quaternary nitrogen capable of carrying a positive charge under the conditions of use of the topical compositions. The beads will have surface charge densities ranging from 0.1 to 10 milliequivalent/gram (meq/gm) capacity for hydrogen ion in water (determined by conventional acid-base titration), usually from 0.2 to 9 meq/gm, more usually from 0.5 to 5 meq/gm, and preferably from 0.5 to 3.5 meq/gm.

In their most convenient form, the particles are generally spherical in shape, due to the use of suspension polymerization as a preferred method of preparation. While such microspheres vary widely in size, those falling within the range of 5 to 100 µm (microns) in diameter, preferably from 10 to 40 µm (microns), will provide the best results. Microspheres within these size ranges are appealing from an aesthetic point of view by imparting a smooth feel to the touch.

The pore dimensions within the spheres may also vary widely, with optimum dimensions depending on the chemical characteristics of the polymers used, as well as the diffusive characteristics of the impregnant. Different systems will thus call for different optimum ranges of pore volume distribution to obtain the most desirable properties for the overall formulation. In general, however, the best results are obtained with total pore volumes ranging from 0.01 to 4.0 cc/g, preferably from 0.1 to 2.00 cc/g, surface areas ranging from 1 to 500 m²/g, preferably from 20 to 200 m²/g, and the average pore diameters ranging from 0.001 to 3.0 µm (micron), preferably from 0.003 to 1.0 µm (micron). Following conventional methods of measuring and expressing pore sizes, the pore diameters are calculated from the measurement of the surface area by B.E.T. nitrogen analysis (Brunaer et al. (1938) J. Am. Chem. Soc. 60:309-316) and from the measurement of the pore volumes by the mercury intrusion method.

The particles are conveniently formed as microspheres by suspension polymerization in a liquid-liquid system. In general, a solution containing the desired monomers, a polymerization catalyst (if used), and an inert fluid (porogen) is formed in a first liquid phase, where the porogen is miscible with the first fluid phase but immiscible with a second liquid phase. The solution is then suspended in the second liquid phase which is immiscible with the first liquid phase. In the case of organic-soluble monomers, e.g., vinyl pyridine and its derivatives, the first liquid phase will usually be an organic solvent capable of dissolving the monomers but which is immiscible with water, and the second liquid phase will be water. In the case of water-soluble monomers, e.g., quaternized acrylate and methacrylate derivatives, the first liquid phase will be aqueous while the second phase will be a hydrophobic organic solvent.

Once the suspension is established with discrete droplets of a desired size, polymerization is effected (typically by activating the reactants by either increased temperature or irradiation). After polymerization is complete, the resulting rigid beads are recovered from the suspension. The beads are, at this point, substantially rigid pore structures, the polymer having formed around the inert fluid thereby forming the pore network. The fluid has accordingly served as porogen, or pore-forming agent, and occupies the pores of the formed beads. Suitable porogen fluids will be described in more detail hereinafter.

Certain impregnants may serve as porogen and, as previously mentioned, may be entrapped within the porous network of the present invention before or after the protonation steps described above. The critical factor in choosing the impregnant is its electrical charge. That is, in order to preserve the cationic functionality, the impregnant must be substantially neutral. Slightly negative or positive substances may be used; however, their entrapment must not neutralize or otherwise affect the surface charge of the bead.

Where the impregnant so selected serves as the porogen, the porous beads recovered from the suspension immediately after polymerization are substantially ready for use, following removal of surface moisture, and any further processing steps of this nature, including protonation. In these cases, microsphere formation and incorporation of the impregnant is performed in a single step. This may accordingly be termed a one-step procedure. Those impregnants which are capable of serving as porogens will be liquid impregnants meeting the following criteria:
1) They are either wholly miscible with a monomer mixture or capable of being made fully miscible by the addition of a minor amount of a solvent which is non-miscible with the second liquid phase;
2) They are immiscible with the second liquid phase (or at most slightly soluble);
3) They are inert with respect to the monomers, and stable when in contact with any polymerization catalyst used and when subjected to any conditions needed to induce polymerization (such as temperature and radiation);
4) They are normally liquids or have melting points below the polymerization temperature. Solids can frequently be converted to liquid form by being dissolved in a solvent or by forming eutectic mixtures; and
5) They are neutral with respect to their electrical charge (or at most either slightly negative or positive).

When using this method, the steps must be performed under an inert atmosphere such as nitrogen. If a polymerization catalyst is used, it must be one which does not oxidize the impregnant, if the latter is susceptible to oxidation. Azo catalysts are examples of such catalysts. Also, polymerization temperatures are being held within a moderate range.

As an alternative to the one-step procedure, the substantially neutral impregnant may be placed inside the pores of preformed dry porous polymer beads. Thus, the product is prepared in two steps, performed in sequence, wherein polymerization is performed first with a substitute porogen which is then removed and replaced by the desired active ingredient. Hence, the porogen and active ingredients are distinct components in this two-step process. Materials suitable as substitute porogens will be substances which meet the five criteria listed above for porogen impregnants.

Preferred among these substances suitable as substitute porogens are hydrocarbons, particularly inert, non-polar organic solvents. Some of the most convenient examples are alkanes, cycloalkanes, and aromatics. Examples of such solvents are alkanes of 5 to 12 carbon atmos, straight or branched chain, cycloalkanes of 5 to 8 carbon atoms, benzene, and alkyl-substituted benzenes such as toluene and the xylenes. Porogens of other types include C₄ - C₂₀ alcohols perfluoro polyethers, and oils. Removal of the porogen may be effected by solvent extraction, evaporation, or similar conventional operations.

A further advantage of the use of this two-step process is that it permits the removal of unwanted species formed within the polymerized structures prior to incorporation of the impregnant. Examples of unwanted species include unreacted monomers, residual catalysts, and surface active agents and/or dispersants remaining on the sphere surfaces. A further advantage of this technique is that it permits one to select the amount and type of porogen as a means of controlling the pore characteristics of the finished bead. One is thus no longer bound by the limitations of the impregnant as it affects the structure of the bead itself. This permits partial, rather than full, filling of the pores with the impregnant, and further control of the pore size and distribution by selection among swelling and non-swelling porogens.

Extraction of the porogen and its replacement with (i.e., impregnation of the dry bead with) the impregnant in the two-step procedure may be effected in a variety of ways, depending on the chemical nature of the porogen and its behavior in combination with that of the other species present. The beads are first recovered from the suspension by filtration, preferably using vacuum filtration apparatus (such as a Buchner funnel). The beads are then washed with an appropriate solvent to remove organic species not bound to the polymer, including surfactants having deposited on the bead surfaces form the aqueous phase, unreacted monomers and residual catalysts, and the porogen itself. An example of such a solvent is isopropanol, either alone or in aqueous solution. Once washing is complete, the solvent itself is removed by drying, preferably in a vacuum.

In certain cases, an alternative method of extraction may be used -- i.e., where the porogen, unreacted monomer and water will form an azeotrope. In these cases, steam distillation is an effective way of extracting porogen from the beads. This again may be followed by drying under vacuum.

Assuming that the beads were already protonated, or that protonation will follow entrapment (as described in more detail hereinbelow), once the beads are rendered dry and free of the substitute porogen and any unwanted organic materials, they are impregnated with the impregnant according to conventional techniques. The most convenient such technique is contact absorption. Solid active ingredients are first dissolved in a solvent, and the resulting solution is absorbed by the beads. The solvent may either be retained in the finished product or removed by conventional means such as evaporation or extraction using a further solvent. For those solid ingredients having limited solubility in a particular solvent, high contents in the finished bead can be attained by repeated absorptions each followed by solvent removal.

The polymerization process and the various parameters and process conditions involved in the polymerization can be selected and adjusted as a means of controlling the pore characteristics and consequently the capacity and release characteristics of the ultimate product. For example, proper selection of the crosslinking means, the amount and type of crosslinking agent, and the amount and type of porogen are means of attaining such control. Certain polymerization conditions may also be varied to such effect, including temperature, degree of radiation where used, degree of agitation and any other factors affecting the rate of the polymerization reaction.

Crosslinking in the polymer formation is a major means of pore size control. Monomers which may be polymerized to produce crosslinked polymer beads in accordance with the present invention include polyethylenically unsaturated monomers, i.e., those having at least two sites of unsaturation, and monoethylenically unsaturated monomers in combination with one or more polyethylenically unsaturated monomers. In the latter case, the percentage of crosslinking may be controlled by balancing the relative amounts of monoethylenically unsaturated monomer and polyethylenically unsaturated monomer. Usually, such systems will include a single monoethylenically saturated monomer and a single polyethylenically unsaturated monomer, although it will be possible to add additional compatible monomers of each type to the system, if desired. For a discussion of the preparation of such copolymer systems, see Guyot and Bartholin, *Design and Properties of Polymers as Materials for Fine Chemistry*, Prog. Polym. Ed. (1982) Vol. 8, pp 303-307.

Monoethylenically unsaturated monomers which may be used as part of the monoethylenically unsaturated monomer content of the polymer delivery system include ethylene, propylene, isobutylene, diisobutylene, styrene, ethylvinylbenzene, vinylbenzene chloride, vinyl pyridine and its derivatives, vinyltoluene, and dicyclopentadiene; esters of acrylic and methacrylic acid, including the methyl, ethyl, propyl, isopropyl, butyl, sec-butyl, tert-butyl, amyl, hexyl, octyl, ethylhexyl, decyl, dodecyl, cyclohexyl, isobornyl, phenyl, benzyl, alkylphenyl, ethoxymethyl, ethoxyethyl, ethoxypropyl, propoxymethyl, propoxyethyl, propoxypropyl, ethoxyphenyl, ethoxybenzyl, and ethoxycyclohexyl esters; vinyl esters, including vinyl acetate, vinyl propionate, vinyl butyrate and vinyl laurate; vinyl ketones, including vinyl methyl ketone, vinyl ethyl ketone, vinyl isopropyl ketone, and methyl isopropenyl ketone; vinyl ethers, including vinyl methyl ether, vinyl ethyl ether, vinyl propyl ether, and vinyl isobutyl ether; vinyl compounds containing silicon and other metals, such as vinyl siloxanes, and the like. Moreover, polyethylenically unsaturated monomers which ordinarily act as though they have only one unsaturated group, such as isopropene, butadiene and chloroprene, may also be used as part of the monoethylenically unsaturated monomer content.

Polyethylenically unsaturated crosslinking monomers suitable for preparing such polymer beads include diallyl phthalate, ethylene glycol diacrylate, ethylene glycol dimethacrylate, trimethylolpropanetrimethacrylate, divinylsulfone; polyvinyl and polyallyl ethers of ethylene glycol, of glycerol, of pentaerythritol, of diethyleneglycol, of monothio- and dithioderivatives of glycols, and of resorcinol; divinylketone, divinylsulfide, allyl acrylate, diallyl maleate, diallyl fumarate, diallyl succinate, diallyl carbonate, diallyl malonate, diallyl oxalate, diallyl adipate, diallyl sebacate, divinyl sebacate, diallyl tartrate, diallyl silicate, triallyl tricarballylate, triallyl aconitate, triallyl citrate, triallyl phosphate, divinyl naphthalene, divinylbenzene, trivinylbenzene; alkyldivinylbenzenes having from 1 to 4 alkyl groups of 1 to 2 carbon atoms substitute on the benzene nucleus; alkyltrivinylbenzenes having 1 to 3 alkyl groups of 1 to 2 carbon atoms substitute on the benzene nucleus; trivinylnaphthalenes, polyvinylanthracenes, and water-soluble acrylates and methacrylates (as specifically set forth below).

At least a portion of the monomer content will comprise protonatable functionalities which are capable of retaining a positive charge under the conditions of use. Such protonable functionalities may be present on the monoethylenically unsaturated monomers, the polyethylenically unsaturated monomers, or both, where suitable functionalities include pyridine and ammonium. Exemplary monomers include vinyl pyridines, such as 2-vinyl pyridine, 4-vinyl pyridine, 3-methyl-2-vinyl pyridine, 4-methyl-2-vinyl pyridine, 6-methyl-2-vinyl pyridine, 3-ethyl-2-vinyl pyridine, 5-ethyl-2-vinyl pyridine, 2-methyl-3-vinyl pyridine, 2-methyl-4-vinyl pyridine, 2-methyl-5-vinyl pyridine, and 2-ethyl-5-vinyl pyridine, as well as water-soluble acrylated and methacrylates, such as methacrylamidopropylhydroxyethyldimethylammonium acetate, methacrylamidopropyltrimethylammonium chloride, and the quaternization products of dimethylaminoethylmethacrylate and dimethyl sulfate, diethylaminoethylacrylate and dimethyl sulfate, vinylbenzyl chloride and divinylbenzene, and vinylbenzyl and ethylene glycol dimethacrylate.

When using the water-soluble acrylate and methacrylate monomers, it is necessary that all monomers employed be water soluble. Suitable polyethylenically unsaturated monomers (required for cross-linking) include N,N'-methylenebisacrylamide; N,N'-nonamethylenebisacrylamide, and alkoxylated water soluble triacrylate. The inverse suspension polymerization protocol described above for water-soluble quartenized monomers will be employed. The microspheres produced from water soluble quartenized monomers are generally non-rigid hydrogels which are useful for absorbing polar (water and alcohol) soluble materials, such as hydroquinones, methyl salicylate, insect repellants (in alcohols), sunscreens (in alcohol).

The preferred polymer bead will be free from reactive groups which will interact with the porogen and the active ingredient which is ultimately incorporated in the composition. In particular, the beads should be free from reactive amino, hydroxyl, carboxylic, and other reactive functionalities. Such beads will not readily undergo unwanted reactions, will be stable over a pH range from about 1 to about 14, will resist moderate oxidation and reduction, will be stable at temperatures ranging from about 90°C and below, will resist attack by moisture, and will have a relatively long shelf life.

Preferred polymer beads are formed by the copolymerization of 4-vinylpyridine and ethylene glycol dimethacrylate, 4-vinylpyridine and divinylbenzene, 2-vinylpyridine and divinylbenzene, 2-vinylpyridine and ethylene glycol dimethacrylate, ethyl methyl vinylpyridine and divinylbenzene, and ethyl methyl vinylpyridine and ethylene glycol dimethacrylate. Of these systems, 4-vinylpyridine and divinylbenzene is particularly preferred, while the copolymer of 4-vinylpyridine and ethylene glycol dimethacrylate is even more particularly preferred.

The polymer beads will have greater than 10% crosslinking, preferably from 10% to 80% crosslinking, and most preferably from 20% to 60% crosslinking. The percentage crosslinking is defined among those skilled in the art as the weight of polyethylenically unsaturated monomer or monomers divided by the total weight of monomer, including both polyethylenically unsaturated and monoethylenically unsaturated monomers. Usually, the monoethylenically unsaturated monomer will be present at from 20% to 80% of the monomer mixture, preferably 40%, with the polyethylenically unsaturated monomer forming the remainder of the mixture.

Protonation of the polymeric beads may be performed either before or after entrapping the desired impregnant within the porous network. One way of obtaining the cationic beads is, for example, protonating the beads thus recovered from the suspension with an acid medium. In particular, to obtain the positive charge on the surface of the beads, an acid wash such as, for example, a 3% aqueous hydrochloride solution, is performed after the beads are recovered from the polymerization step. Excess acid is removed with a second hydrochloride solution having a pH ranging from 1 to 4; preferably, however, pH 3.

Alternatively, the beads may be protonated with a pH 3 buffered rinse, comprising 0.1N potassium hydrogen phthalate, 0.1N HCl and deionized water. Use of this buffered rinse does not require removal of excess acid, instead the beads so treated are directly filtered and dried.

Once the microspheres are formed and dried, they may be impregnated with the impregnant by contact absorption (this step may be performed either before or after protonation). As an option, the impregnant may be used in the form of a solution in a suitable organic solvent for purposes of decreasing viscosity and facilitating absorption, decreasing potency. Examples of such solvents are liquid petrolatum, ether, petroleum ether, alcohols including methanol, ethanol and higher alcohols, aromatics including benzene and toluene, alkanes including pentane, hexane and heptane, ketones including acetone and methyl ethyl ketone, chlorinated hydrocarbons including chloroform, carbon tetrachloride, methylene chloride and ethylene dichloride, acetates including ethyl acetate, and oils including isopropyl myristate, diisopropyl adipate and, mineral oil. After absorption of the solution, the solvent can be evaporated or, if desired, retained inside the pores together with the impregnant. Other formulating materials, such as carriers or adjuvants such as fragrances, preservatives, antioxidants, and other emollients can also be present, and will be incorporated into and onto the beads together with the impregnants and any other materials present.

Substances incorporated in the cationic polymer bead delivery system of the present invention may be used individually or may be combined to achieve a desired effect. The impregnant, whether it be pure active substance, a mixture of active substances or a solution of active substance(s), will generally comprise between 5% and 65% of the total weight of the impregnated beads. When the active substance is particularly potent, it will generally be in the form of a dilute solution, and the weight percent of the active ingredient itself may range as low as 0.01% based on the total weight of the impregnated beads.

Suitable impregnants include a wide variety of active substances intended for topical application, comprising cosmetic, therapeutic, and other uses. Specific substances include ultraviolet absorbing substances (sunscreens), steroids, insect repellants, retinoic acid, fragrances, minoxidil and emollients. Specific methods for incorporating such substances in polymer bead delivery systems are taught in copending US application serial numbers 091,647 (EP-A-0306236) and 112,971 (EP-A-313380).

Once the beads have been prepared, by either the one-step or two-step procedures described above, they are incorporated in a carrier or vehicle or in virtually any type of product, provided that it is incapable of neutralizing the surface charge on the bead surface, and has at least a slightly acidic pH, preferably being below pH 6, more preferably having a pH in the range from 3 to 4.

The impregnated beads may be incorporated in fluid or solid compositions or preparations of the type commonly used for skin treatment, including gels, creams, lotions, ointments, sprays, powders, oils and sticks. Appropriate vehicles for particular areas or methods of application will be readily apparent to those skilled in the art. For instance, the compositions of the present invention, particularly the UV absorbing compositions, will be incorporated in other products in order to impart cosmetic as well as sunscreen properties. For example, the UV absorbing compositions of the present invention are ideally suited for combining with e.g. make-up foundations, suntan preparations, wherein high adsorption and water-repulsion of the final production is sought.

In general, the compositions and formulations of the present invention are utilized by topical application to keratinic material, particularly human skin and hair. The cationic surface charge on the individual polymeric particles promotes adhesion of the compositions to the skin and hair, enhancing the persistence of the active substance which is being applied.

The following are some of the considerations specific to various particular types of impregnants, plus examples of preparation and utility. The examples are offered solely for purposes of illustration. All parts and percentages are by weight, unless otherwise stated.

### EXPERIMENTAL

### EXAMPLE I

This example illustrates the preparation of 4-vinylpyridine/ethyleneglycoldimethacrylate polymeric beads. The procedure is set forth below:
A 1000 ml four-necked reaction flask equipped with a motorized stirrer, reflux condenser, thermometer, and nitrogen inlet was evaluated and purged with nitrogen. 300 parts of deionized water, 2.5 parts gum arabic and 2.5 parts lignosulfonate under the trademark Marasperse N-22 (Reed Lignin), were added to the reaction flask. The mixture was heated, with stirring, in an oil bath at about 50°C until the dispersants (gum arabic and Marasperse N-22) dissolved to form an aqueous phase.

To this mixture was added a solution of 40 parts of 4-vinylpyridine, 60 parts ethyleneglycoldimethacrylate, 0.80 parts benzoyl peroxide (70% active ingredient and 30% water), and 50 parts toluene (porogen). The aqueous phase and organic solution were agitated by stirring at a rate (approx. 900 rpm) adjusted to give a plurality of droplets having a droplet diameter in the range of 5 to 100 µm (microns), as determined by visual observation of a sample of the droplets with an optical microscope with the droplets being stabilized by the dispersants.

The reaction mixture was then heated to 60 to 65°C for 20 minutes, and heating was continued for another 8 hours at 74-76°C to form porous beads of crosslinked 4-vinylpyridine/ethyleneglycoldimethacrylate having toluene entrapped within the network of pores. The reaction mixture was then cooled to room temperature and the porous polymeric beads were removed from the reaction flask by filtration. The filtered beads were washed initially twice with one liter portions of deionized water to remove the dispersants, followed by two washes with one liter portions of isopropanol to remove any residual, unreacted monomers and the toluene. The beads were then dried in an oven at 80 to 90°C for about 8 hours.

The yield was 87.0g of opaque beads. The average particle diameter of these beads was 25 µm (microns), as measured by Sedimentation Micromeritics Instrument Co. The particle diameter determination method is described in detail in the *Microsizer 5300 Particle Size Analyzer Instruction Manual*, (1984) associated with the instrument.

The surface area of a sample of the purified beads was determined by the B.E.T. Nitrogen Analysis method to be 11.05 m²/g, while the pore volume was determined by the mercury intrusion method to be 0.14 ml/g.

### EXAMPLE II

This example illustrates the protonation of the 4-vinylpyridine/ethyleneglycoldimethacrylate polymeric beads of Example I.

To a 1000 ml flask was added 80.0g of preformed porous beads from Example I and 300 ml of 3% aqueous solution of hydrochloride.

After stirring the slurry for three hours, the porous polymeric cationic beads were filtered and washed with a dilute hydrochloride solution, pH 3, to remove excess 3% acid solution from the polymeric beads. The beads were then dried in an oven at 75°C for 8 to 10 hours. Hydrogen ion (H⁺) capacity in water was measured to be 0.78 meq/g.

### EXAMPLE III

This example illustrates the preparation and protonation of 4-vinylpyridine/divinylbenzene polymeric beads. The procedure is set forth below:

The reaction apparatus was prepared as in Example I. To the reaction flask was added 600 parts deionized H₂0, 6.0 parts gum arabic, and 6.0 parts Marasperse N-22. The aqueous solution was stirred at room temperature until all solids were dissolved.

To the flask was added an organic solution containing 35 parts 4-vinylpyridine, 65 parts divinylbenzene (55% divinylbenzene, 45% ethylvinylbenzene), 100 parts isobutanol, and 1.0 part 2, 2'-azobis (2-methylbutanenitrile) available from the DuPont Co. under the tradename VAZO 67. The reaction mixture was agitated at approximately 1300 rpm until droplets were formed as in Example I. The reaction mixture was then heated to 75°C at which point agitation was reduced to 800 rpm. The reaction was allowed to continue for 8 hours at this temperature.

Opaque porous beads were collected by filtration and washed three times with 500 ml portions of deionized water. Protonation was effected by stirring the beads in 500 ml of 0.1N HCl solution for 30 minutes. The beads were filtered and washed with a dilute hydrochloride solution, pH 3, to remove excess acid. Residual monomers and porogen were removed as in Example I. A dry powder was obtained after drying the beads in an 80 to 90°C oven for approximately 8 hours. The yield was 93 g. The average particle diameter, surface area, and pore volume were 36 micron, 2.21 m²/g, and 0.073 ml/g, respectively.

### EXAMPLE IV

This example illustrates an alternative method for protonizing polymeric beads, using a buffered rinse. The procedure is set forth below:
To a 1000 ml beaker was added 100g of preformed porous beads from Example I, 250 ml of a pH 3.0 buffer (consisting of 500 parts 0.1N potassium hydrogen phthalate and 223 parts 0.1N HCl), and 250 ml deionized H₂O. This mixture was stirred for 30 minutes, then filtered. The quaternized beads were dried in an 80 to 90°C oven for 6 to 10 hours.

### EXAMPLE V

This example illustrates the preparation of 4-vinylpyridine/ethyleneglycoldimethacrylate beads using xylene as the porogen. The procedure is set forth below:
A 1000 ml reaction flask was charged with an aqueous dispersion solution as described in Example I. An organic solution was prepared as in Example I, with the exception of using 50 parts xylenes (a mixture of ortho, meta and para isomers) as porogen rather than toluene. The reaction was agitated at 1300 rpm until droplet sizes ranged from 10 to 60 µm (microns). The reaction was then heated to 65°C and maintained at this temperature for 20 minutes. Agitation was reduced to 800 rpm, and the reaction was heated to 75°C. The reaction was allowed to continue at this temperature for 8 hours.

The porous beads were collected by filtration and rinsed with 500 ml deionized H₂O. The beads were then quaternized with a pH 3.0 buffer as described in Example IV. The residual monomers and porogen were removed by rinsing the beads three times with 500 ml portions of acetone. After drying approximately 8 hours in a 80 to 90°C oven, 61g of beads were obtained. The average particle diameter, surface area, and pore volume were 22.5 microns, 3.03 m²/g, and 0.68 ml/g, respectively.

### EXAMPLE VI

This example illustrates the substitution of an ultraviolet absorbing substance (sunscreen) in the cationic beads of Example II. The procedure is set forth below:
An 18.0 parts portion of the porous cationic polymeric beads obtained from Example II was mixed at room temperature with 30 parts of isopropanol in a glass flask with an agitator. Then 12.0 parts of a sunscreen mixture containing 7 parts octyldimethyl PABA and 2 parts Oxybenzone were added slowly. The resulting suspension was stirred for about 20 minutes. The solvent was then allowed to evaporate to dryness in a fume hood at room temperature for 24 hours. Approximately 40% of the sunscreen mixture was entrapped within the pores of the cationic polymeric beads.

### EXAMPLE VII

The adherence and retention of the 4-vinylpyridine/ethyleneglycoldimethacrylate (4-VP/EGDMA) copolymer beads to human skin was investigated in two human subjects. Unprotonated and protonated 4-VP/EGDMA beads were prepared as described in Examples I and II, above, and loaded with an oil soluble dye (Oil Red EGN). The dye was extracted from a small sample of beads and the percentage loading (wt. dye/wt. dye + beads x 100) was quantitated using a spectrophometric method. The unprotonated beads were found to have 0.9% loading while the prontonated beads were found to have 1.0% loading.

Measured amounts of each bead preparation (one polymer on each hand) were applied to the marked areas of the forearms of the subjects covering an area of 6.14 cm². The arms were then immersed in water for five seconds and then removed. This cycle was repeated five times (the hands were not dried between dips), and the polymer retained on the skin was recovered by washing with surfactant solution. The amount of beads retained was determined by extracting out the dye, quantitating it using a spectrophotometric method and correlating the amount of dye extracted to the amount of polymer. The results are present in Table 1.

**TABLE 1**

| Polymer Beads | Subject | Amount Applied/cm² (mg) | Amount Retained (mg)/cm² | % Retained |
|---|---|---|---|---|
| Unprotonated | 1 | 1.86 | 0.67 | |
| | 2 | 1.89 | 0.61 | 34.26 |
| Protonated | 1 | 1.61 | 0.81 | |
| | 2 | 1.86 | 1.25 | 58.93 |

The results demonstrate that a greater amount of the protonated polymer was retained on the skin compared to the unprotonated polymer.

## Claims

1. A topical composition comprising solid beads containing a network of pores open to the exterior of said beads and an impregnant retained inside said pore network and a carrier suitable for topical application characterised in that the bead material is protonated to have a cationic surface charge sufficient to promote adhesion to keratinic materials, and the carrier has a pH below 6.

2. A topical composition according to claim 1, wherein the surface charge is in the range 0.1 to 10 meq/gm hydrogen ion capacity.

3. A topical composition according to claim 2, wherein the surface charge is in the range 0.5 to 3.5 meq/gm hydrogen ion capacity.

4. A topical composition according to claim 1, wherein the impregnant is selected from the group consisting of ultraviolet absorbing substances, steroids, insect repellants, retinoic acid, fragrances, minoxidil, and emollients.

5. A topical composition according to any one of the preceding claims wherein the pH of the carrier is 3 to 4.

6. A topical composition according to Claim 1 wherein said beads are substantially non-collapsible polymeric beads, each defining a network of internal pores open to the exterior surface of said polymeric beads, wherein the polymer at the exterior surface of said beads is protonated and said beads are substantially spherical in shape and have an average diameter of 1 µm (micron) to 100 µm (microns), a total pore volume of 0.01 cc/g to 4.0 cc/g, an average surface area of 1 m²/g to 500 m²/g, an average pore diameter of 0.001 µm (micron) to 3.0 µm (micron), a crosslinking density of at least 10%, and a surface charge density in the range from 0.1 to 10 meq/gm hydrogen ion capacity.

7. A topical composition according to any one of the preceding claims wherein the beads are a copolymer of 4-vinyl pyridine and ethylene glycol dimethacrylate, of 4-vinyl pyridine and divinylbenzene, or of quartenized vinylbenzyl chloride and (i) divinylbenzene or (ii) dimethylmethacrylate.

8. A method for preparing a topical composition, the method comprising:
(a) combining a monomer solution capable of forming a crosslinked polymer with a porogen in a first liquid phase, wherein at least a portion of the monomers carry a protonatable functionality;
(b) dispensing the first liquid phase in a second liquid phase immiscible with the first liquid phase whereby droplets of the first liquid phase are formed;
(c) polymerizing the monomers within the droplets to form polymeric beads, each bead having a network of internal pores open to the exterior surface of said polymeric beads;
(d) recovering the polymeric beads from said liquid phases;
(e) protonating the beads to impart a positive surface charge and
(f) incorporating the beads in a liquid carrier or vehicle of pH lower than 6.

9. A method according to claim 8, wherein the monomers are organic-soluble and the first liquid phase is organic immiscible with water while the second liquid phase is water.

10. A method according to claim 9, wherein the monomers include vinyl pyridine.

11. A method according to claim 10, wherein the monomers are water-soluble and the first liquid phase is aqueous and the second liquid phase is a hydrophobic organic solvent.

12. A method according to claim 11, wherein the monomers include water quarternized acrylates and methacrylates.

13. A method according to claim 8, wherein the beads are protonated by washing in an acidic medium.

14. A method according to claim 8, wherein the porogen is an active topical ingredient.

15. A method as in claim 14, wherein the active topical ingredient is an ultraviolet absorbing substance, steroid, insect repellant, retinoic acid, fragrance, minoxidil, or emollient.

16. A method according to claim 8, further comprising:
extracting the porogen from the beads; and
absorbing an active topical ingredient into the pores of the beads.

17. A method according to claim 16, wherein the active topical ingredient is selected from the group consisting of ultraviolet absorbing substances, steroids, insect repellants, retinoic acid, fragrances, minoxidil, and emollients.

18. A method of preparing a topical composition, said method comprising infusing an active topical ingredient into preformed porous polymeric particles, wherein said particles are characterized by a positive charge on their surface, said positive charge being sufficient to promote adhesion of the particles to keratinic material, and incorporating the particles into a liquid carrier or vehicle of pH below 6.

19. A method for topically applying a cosmetically active ingredient to a keratinic material, said method comprising applying to said keratinic material a topical composition according to any one of claims 1 to 7.

## Patentansprüche

1. Topische Zusammensetzung umfassend feste Perlen, die ein zur Außenfläche der genannten Perlen hin offenes Porennetzwerk, wobei innerhalb des Porennetzwerks ein Imprägnierungsmittel gehalten ist, und einen zur topischen Anwendung geeigneten Träger, dadurch gekennzeichnet, daß das Perlenmaterial protoniert ist, sodaß es eine kationische Oberflächenladung aufweist, die ausreicht, um die Haftung an Keratinmaterialien zu fördern, und der Träger einen pH-Wert von weniger als 6 aufweist.

2. Topische Zusammensetzung nach Anspruch 1, worin die Oberflächenladung im Bereich von 0,1 bis 10 mäq/gm Wasserstoffionenkapazität liegt.

3. Topische Zusammensetzung nach Anspruch 2, worin die Oberflächenladung im Bereich von 0,5 bis 3,5 mäq/gm Wasserstoffionenkapazität liegt.

4. Topische Zusammensetzung nach Anspruch 1, worin das Imprägniermittel aus der Gruppe ausgewählt ist, die aus UV-absorbierenden Substanzen, Steroiden, Insektenrepellents, Retinoesäure, Duftstoffen, Monoxidil und Weichmachern besteht.

5. Topische Zusammensetzung nach einem der vorhergehenden Ansprüche, worin der pH-Wert des Trägers 3 bis 4 beträgt.

6. Topische Zusammensetzung nach Anspruch 1, worin genannte Perlen im wesentlichen nicht kollabierbare Polymerperlen sind, von denen jede ein Netzwerk innerer Poren definiert, das zur Außenfläche der genannten Polymerperlen hin offen ist, worin das Polymer an der Außenfläche der genannten Perlen protoniert ist und die genannten Perlen im wesentlichen kugelförmig sind und einen mittleren Durchmesser von 1 µm bis 100 µm, ein Gesamtporenvolumen von 0,01 cm²/g bis 4,0 cm²/g, eine mittlere Oberfläche von 1 m²/g bis 500 m²/g, einen mittleren Porendurchmesser von 0,001 µm bis 3,0 µm, eine Vernetzungsdichte von zumindest 10% und eine Oberflächenladungsdichte im Bereich von 0,1 bis 10 mäq/ gm Wassertoffionenkapazität aufweisen.

7. Topische Zusammensetzung nach einem der vorhergehenden Ansprüche, worin die Perlen aus einem Copolymer von 4-Vinylpyridin und Äthylenglykoldimethacrylat, von 4-Vinylpyridin und Divinylbenzol, oder von quaternisiertem Vinylbenzylchlorid und (i) Divinylbenzol oder (ii) Dimethylmethacrylat bestehen.

8. Verfahren zur Herstellung einer topischen Zusammensetzung, wobei das Verfahren umfaßt:
(a) Kombinieren einer Monomerlösung, die fähig ist, ein vernetztes Polymer zu bilden, mit einem Porogen in einer ersten flüssigen Phase, worin zumindest ein Anteil der Monomeren eine protonierbare Funktionalität trägt;
(b) Dispergieren der ersten flüssigen Phase in einer zweiten flüssigen Phase, die mit der ersten flüssigen Phase unvermischbar ist, wodurch Tröpfchen der ersten flüssigen Phase gebildet werden;
(c) Polymerisieren der Monomeren innerhalb der Tröpfchen zur Bildung von Polymerperlen, wobei jede Perle ein Netzwerk von Innenporen aufweist, das zur Außenfläche der genannten Polymerperlen hin offen ist;
(d) Gewinnen der Polymerperlen aus den genannten flüssigen Phasen;
(e) Protonieren der Perlen zur Verleihung einer positiven Oberflächenladung und
(f) Einarbeiten der Perlen in einen flüssigen Träger oder ein flüssiges Vehikel von einem pH-Wert von weniger als 6.

9. Verfahren nach Anspruch 8, worin die Monomeren in organischen Medien löslich sind und die erste flüssige Phase ein mit Wasser unvermischbares organisches Medium ist, während die zweite flüssige Phase Wasser ist.

10. Verfahren nach Anspruch 9, worin die Monomeren Vinylpyridin umfassen.

11. Verfahren nach Anspruch 8, worin die Monomeren wasserlöslich sind, die erste flüssige Phase wässerig ist und die zweite flüssige Phase ein hydrophobes organisches Lösungsmittel ist.

12. Verfahren nach Anspruch 11, worin die Monomeren wasserquaternisierte Acrylate und Methacrylate enthalten.

13. Verfahren nach Anspruch 8, worin die Perlen durch Waschen in einem sauren Medium protoniert werden.

14. Verfahren nach Anspruch 8, worin das Porogen ein topisches Wirkingrediens ist.

15. Verfahren nach Anspruch 14, worin das topische Wirkingrediens eine UV-absorbierende Substanz, ein Steroid, Insektenrepellent, Retinoesäure, ein Duftstoff, Monoxidil oder ein Weichmacher ist.

16. Verfahren nach Anspruch 8, weiters umfassend:
Extrahieren des Porogens aus den Perlen und Absorbieren eines topischen Wirkingrediens in die Poren der Perlen.

17. Verfahren nach Anspruch 16, worin das topische Wirkingrediens aus der Gruppe ausgewählt ist, die aus UV-absorbierenden Substanzen, Steroiden, Insektenrepellents, Retinoesäure, Duftstoffen, Minoxidil und Weichmachern besteht.

18. Verfahren zum Herstellen einer topischen Zusammensetzung, wobei das genannte Verfahren das Infundieren eines topischen Wirkingrediens in vorgeformte poröse Polymerteilchen, wobei die genannten Teilchen durch eine positive Ladung an ihrer Oberfläche gekennzeichnet sind und die genannte positive Ladung ausreicht, um die Haftung der Teilchen an Keratinmaterial zu fördern, und das Einarbeiten der Teilchen in einen flüssigen Träger oder ein flüssiges Vehikel mit einem pH-Wert von weniger als 6 umfaßt.

19. Verfahren zum topischen Auftragen eines kosmetisch aktiven Ingrediens auf ein Keratinmaterial, wobei das genannte Verfahren das Auftragen einer topischen Zusammensetzung nach einem der Ansprüche 1 bis 7 auf das genannte Keratinmaterial umfaßt.

## Revendications

1. Composition topique comprenant des billes solides contenant un réseau de portes ouverts à l'extérieur desdites billes et un composé d'imprégnation retenu à l'intérieur dudit réseau de pores et un porteur approprié pour application tonique caractérisée en ce que le matériau des billes est protoné pour avoir une charge de surface cationique suffisante pour promouvoir l'adhésion de matériau kératinique, et en ce que le porteur a un pH en dessous de 6.

2. Composition topique selon la revendication 1, dans laquelle la charge de surface est dans l'intervalle de 0,1 à 10 meq/g de capacité d'ion hydrogène.

3. Composition topique selon la revendication 2, dans laquelle la charge de surface est dans l'intervalle de 0,5 à 3,5 meq/g de capacité d'ion hydrogène.

4. Composition topique selon la revendication 1, dans laquelle le composé d'imprégnation est choisi parmi le groupe consistant des substances absorbant les ultraviolets, des stéroïdes, des répulsifs d'insectes, de l'acide rétinoïque, des parfums, du minoxidile, et des émollients.

5. Composition topique selon l'une quelconque des revendications précédentes, dans laquelle le pH du porteur est de 3 à 4.

6. Composition topique selon la revendication 1, dans laquelle lesdites billes sont substantiellement susceptibles de ne pas s'écrouler, chacune définissant un réseau de pores internes ouverts à la surface extérieure desdites billes polymériques, où le polymère à la surface extérieure desdites billes est protoné et lesdites billes sont de forme substantiellement sphérique et ont un diamètre moyen de 1µm (micron) à 100µm (microns), un volume de pores total de 0,01cc/g à 4,0cc/g, une surface spécifique moyenne de 1m²/g à 500m²/g, un diamètre de pores moyen de 0,001µm (micron) à 3,0µm (microns), une densité de réticulation d'au moins 10%, et une densité de charge de surface dans l'intervalle de 0,1 à 10meq/g de capacité d'ion hydrogène.

7. Composition topique selon l'une quelconque des revendications précédentes dans laquelle les billes sont un copolymère de 4-vinyl-pyridine et d'éthylène glycol diméthacrylate, de 4-vinyl-pyridine et de divinylbenzyène, ou de chlorure de vinylbenzyle quaternisé et (i) de divinylbenzène ou (ii) de diméthylméthacrylate.

8. Méthode pour préparer une composition topique, la méthode comprenant :
(a) combinaison d'une solution de monomères capables de former un polymère réticulé avec un porogène dans une première phase liquide, où au moins une partie des monomères portent une fonctionnalité protonable;
(b) mettre la première phase liquide dans une seconde phase liquide non miscible avec la première phase liquide ce par quoi des gouttes de la première phase liquide sont formées;
(c) polymérisation des monomères dans les gouttelettes pour former des billes polymériques, chaque bille ayant un réseau de pores internes ouverts à l'extérieur de la surface extérieure desdites billes polymériques;
(d) récupération des billes polymériques desdites phases liquides;
(e) protonation des billes pour impartir une charge de surface positive et
(f) incorporation des billes dans un véhicule ou porteur liquide de pH inférieur à 6.

9. Méthode selon la revendication 8, dans laquelle les monomères sont solubles dans un milieu organique et la première phase liquide est un composé organique non miscible avec l'eau tandis que la seconde phase liquide est l'eau.

10. Méthode selon la revendication 9, dans laquelle le monomère inclut de la vinyl-pyridine.

11. Méthode selon la revendication 10, dans laquelle les monomères sont solubles dans l'eau et la première phase liquide est aqueuse et la seconde phase liquide est un solvant organique hydrophobe.

12. Méthode selon la revendication 11, dans laquelle les monomères incluent des acrylates quaternisés dans l'eau et des méthacrylates.

13. Méthode selon la revendication 8, dans laquelle les billes sont protonées par lavage dans un milieu acide.

14. Méthode selon la revendication 8, dans laquelle le porogène est un ingrédient actif topique.

15. Méthode selon la revendication 14, dans laquelle l'ingrédient topique actif est une substance absorbant les ultraviolets, un stéroïde, un répulsif d'insecte, l'acide rétinoïque, un parfum, le minoxidile, ou un émollient.

16. Méthode selon la revendication 8, comprenant de plus :
extraction du porogène des billes; et
absorption d'un ingrédient actif topique dans les pores des billes.

17. Méthode selon la revendication 16, dans laquelle l'ingrédient topique actif est choisi dans le groupe consistant des substances absorbants des ultraviolets, des stéroïdes, des répulsifs d'insectes, de l'acide rétinoïque, des parfums, du minoxidile, et des émollients.

18. Méthode pour préparer une composition topique, ladite méthode comprenant la perfusion d'un ingrédient topique actif dans des particules polymèriques poreuses préformées, où lesdites particules sont caractérisées par une charge positive sur la surface, ladite charge positive étant suffisante pour promouvoir l'adhésion des particules à un matériau kératinique, et l'incorporation des particules dans un véhicule ou porteur liquide de pH inférieur à 6.

19. Méthode pour appliquer topiquement un ingrédient actif cosmétiquement à un matériau kératinique, ladite méthode comprenant l'application dudit matériau kératinique à une composition topique selon l'une quelconque des revendications 1 à 7.
